# EUROPÄISCHE PATENTSCHRIFT

(11) **EP 2 187 992 B1**
(45) Veröffentlichungstag und Bekanntmachung des Hinweises auf die Patenterteilung: **01.08.2018**
(21) Anmeldenummer: 08801719.9
(22) Anmeldetag: 26.08.2008
(51) Int. Cl.: A61M 1/34, A61M 1/36, A61M 5/168, A61M 5/145

(54) **VERFAHREN ZUR ÜBERPRÜFUNG UND/ODER ÜBERWACHUNG DER KORREKTEN FUNKTION EINER ZUGABEVORRICHTUNG**
METHOD FOR VERIFYING AND/OR MONITORING THE CORRECT FUNCTION OF A SUPPLY DEVICE
PROCÉDÉ PERMETTANT DE CONTRÔLER ET/OU DE SURVEILLER LE FONCTIONNEMENT CORRECT D'UN DISPOSITIF D'ADDITION

(30) Priorität: 18.09.2007 DE 102007044413
(43) Veröffentlichungstag der Anmeldung: 26.05.2010
(73) Patentinhaber: Fresenius Medical Care Deutschland GmbH, 61352 Bad Homburg (DE)
(72) Erfinder: KOPPERSCHMIDT, Pascal, 97456 Dittelbrunn (DE)
(74) Vertreter: Laufhütte, Dieter
(86) Internationale Anmeldenummer: PCT/EP2008/006990
(87) Internationale Veröffentlichungsnummer: WO 2009/036866

(56) Entgegenhaltungen:
- EP-A- 0 328 162
- WO-A-2007/012915
- DE-C1- 10 159 620
- DE-C1- 10 213 179

## Beschreibung

Die vorliegende Erfindung betrifft ein Verfahren zur Überprüfung und/oder Überwachung der korrekten Funktion einer Zugabevorrichtung eines medizinischen Geräts, wobei das medizinische Gerät einen extrakorporalen Kreislauf aufweist, mit dem die Zugabevorrichtung derart in Verbindung steht, das mittels der Zugabevorrichtung ein Mittel in den extrakorporalen Kreislauf einführbar ist.

Insbesondere kann es sich bei dem medizinischen Gerät dabei um ein Dialysegerät handeln, bei welchem - wie auch bei anderen extrakorporalen Verfahren - dem Blut ein Antikoagulationsmittel wie z. B. Heparin oder Zitrat zugegeben werden muss. Hierzu weist das medizinische Gerät eine Zugabevorrichtung auf, üblicherweise in Form einer Spritzenpumpe, welche das zuzugebende Mittel enthält. Vor Beginn der Behandlung wird die Zugabevorrichtung mit dem extrakorporalen Kreislauf, d. h. mit einem extrakorporalen Schlauchset, verbunden.

Bei Spritzenpumpen wird zunächst eine Spritze mit dem Mittel in die Spritzenpumpe eingelegt und mit dem extrakorporalen Kreislauf verbunden. Zur Zugabe des Mittels aus der Spritze in den extrakorporalen Kreislauf ist ein Stößel vorgesehen, welcher den Spritzenstempel in die Spritze drückt. Der Stößel wird hierzu über eine Spindel und einen Spindelantrieb entsprechend bewegt. Die Überwachung der durch eine solche Zugabevorrichtung verabreichten Dosis erfolgt dabei z. B. während der Dialysebehandlung über die Messung der Stößelverschiebung, z. B. durch die Abtastung mittels einer Widerstandspiste. Hierdurch kann der zurückgelegte Weg des Förderstößels und damit die zugegebene Menge berechnet werden.

Hierdurch wird die korrekte Zugabe aber lediglich auf Seite der Spritzenpumpe überprüft, während eine Sicherstellung, ob die zu infundierende Menge an Antikoagulanzmittel auch wirklich im extrakorporalen Kreislauf ankommt, nicht erfolgt. Zwar wird auch in bekannten Geräten zu Beginn der Behandlung initial überprüft, ob die Spritzenpumpe richtig konnektiert ist. Während der Dauer der Behandlung wäre eine Dekonnektierung oder sonstige Fehlfunktion jedoch nicht automatisch erkennbar.

Insbesondere im Fall einer nicht korrekt eingelegten Infusionsspritze könnte dabei ein initialer Ankoppeltest erfolgreich abgeschlossen werden. Probleme durch die nicht korrekt eingelegte Infusionsspritze, welche sich erst während der Behandlung ergeben und zu einer Abweichung der rezeptierten Infusionsgabe führen, könnten jedoch nicht erkannt werden, da die verabreichte Dosis der Infusion lediglich indirekt über die Stößelbewegung, jedoch nicht direkt über die Stempelbewegung überwacht wird.

Aus der DE 101 59 620 C1 und der DE 102 13 179 C1 sind jeweils Verfahren gemäß dem Oberbegriff von Anspruch 1 bekannt. Dabei wird die Zufuhr von Substitutionsflüssigkeit in einen extrakorporalen Kreislauf überwacht, indem die durch die Substituatpumpe erzeugten Druckpulse überwacht werden. Aus der EP 328 162 A2 ist ein Verfahren zur Fehlererkennung bei einer Infusionspumpe bekannt, bei welchem die von der Infusionspumpe erzeugten Druckpulse analysiert werden. Die dort gezeigten Verfahren sind auf den Einsatz von peristaltischen Pumpen beschränkt.

Aufgabe der vorliegenden Erfindung ist es daher, ein verbessertes Verfahren zur Überprüfung und/oder Überwachung der korrekten Funktion einer Zugabevorrichtung zur Verfügung zu stellen. Insbesondere soll über dieses Verfahren die Möglichkeit gegeben werden, auch während dem laufenden Betrieb die korrekte Funktion der Zugabevorrichtung direkt überprüfen zu können.

Erfindungsgemäß wird diese Aufgabe von einem Verfahren gemäß Anspruch 1 gelöst.

Bei diesem Verfahren wird ein charakteristischer Signalverlauf im extrakorporalen Kreislauf erfasst, wobei die Zugabevorrichtung den charakteristischen Signalverlauf erzeugt. Vorteilhafterweise wird dabei ein Signalverlauf im extrakorporalen Blutkreislauf gemessen und zur Überprüfung und/oder Überwachung der korrekten Funktion der Zugabevorrichtung ausgewertet. Dabei wird ein charakteristischer Signalverlauf erfasst, wenn die Zugabevorrichtung korrekt funktioniert und diesen erzeugt, so dass eine direkte Überprüfung und/oder Überwachung möglich wird. Durch die Überprüfung, ob tatsächlich ein charakteristischer Signalverlauf vorliegt, kann so direkt überwacht werden, ob die Zugabevorrichtung korrekt funktioniert. Ist die Zugabevorrichtung dagegen nicht korrekt angekoppelt oder liegen sonstige Probleme vor, welche die korrekte Zugabe von Flüssigkeit aus der Zugabevorrichtung in den extrakorporalen Kreislauf verhindern, wird in diesem auch kein charakteristischer Signalverlauf erzeugt, so dass die Fehlfunktion der Zugabevorrichtung zuverlässig erkannt werden kann. Hierdurch muss nicht mehr auf die indirekte Überprüfung, z. B. durch die Stößelbewegung einer Spritzenpumpe zurückgegriffen werden, sondern die korrekte Funktion kann direkt gemessen werden. Insbesondere lässt sich der charakteristische Signalverlauf dabei auch während des laufenden Betriebs des medizinischen Geräts erzeugen und erfassen, so dass über das erfindungsgemäße Verfahren eine kontinuierliche Überwachung möglich wird.

Erfindungsgemäß wird dabei einer kontinuierlichen Bewegung der Zugabevorrichtung, über welche das Mittel in den extrakorporalen Kreislauf zugegeben wird, zur Erzeugung des charakteristischen, insbesondere oszillierenden Signalverlaufs eine entsprechende charakteristische, insbesondere oszillierende Bewegung überlagert. Wird also ein charakteristischer Signalverlauf im extrakorporalen Blutkreislauf erkannt, bedeutet dies, dass die Zugabevorrichtung korrekt an den extrakorporalen Kreislauf angekoppelt ist und funktioniert, so dass die durch die kontinuierliche Bewegung der Zugabevorrichtung zugegebene Flüssigkeit auch tatsächlich im extrakorporalen Kreislauf ankommt. Wird dagegen kein charakteristischer Signalverlauf festgestellt, bedeutet dies, dass auch keine Flüssigkeit zugegeben wird.

Vorteilhafterweise ist dabei der charakteristische Signalverlauf ein charakteristischer Druckverlauf. Die Zugabevorrichtung erzeugt hierbei den charakteristischen Druckverlauf im extrakorporalen Kreislauf, welcher dann erfaßt werden kann, um die korrekte Funktion der Zugabevorrichtung zu überprüfen und/oder zu überwachen. Hierdurch ist eine einfache Überprüfung und/oder Überwachung z. B. über einen Drucksensor im extrakorporalen Kreislauf möglich.

Alternativ ist ebenfalls denkbar, dass der charakteristische Signalverlauf in den elektrischen Parametern, insbesondere im Antriebsstrom, einer im extrakorporalen Kreislauf angeordneten Pumpe erfaßt wird, welche Flüssigkeit durch den extrakorporalen Kreislauf pumpt. Auch in diesen elektrischen Parametern lässt sich ein charakteristisches Signal, welches mittels der Zugabevorrichtung erzeugt wird, erfassen. Hierbei würde ein von der Zugabevorrichtung erzeugter charakteristischer Druckverlauf nicht mehr direkt gemessen, sondern indirekt als charakteristischer Signalverlauf in den elektrischen Parametern der im extrakorporalen Kreislauf angeordneten Pumpe erkannt.

Weiterhin vorteilhafterweise weist der erfindungsgemäße charakteristische Signalverlauf ein mehrmaliges abwechselndes Ansteigen und Abfallen des Signals, insbesondere mehrere aufeinander folgende pulsförmige Signale, auf. Hierdurch kann die Überprüfung und/oder Überwachung der korrekten Funktion der Zugabevorrichtung sicher und zuverlässig gewährleistet werden. Insbesondere ist über einen solchen charakteristischen Signalverlauf auch eine Überwachung der korrekten Funktion der Zugabevorrichtung möglich, während das medizinische Gerät in Betrieb ist und der extrakorporale Kreislauf dementsprechend offen ist, so dass ein einzelnes Signal nicht sicher erkannt werden könnte.

Weiterhin vorteilhafterweise weist der charakteristische Signalverlauf dabei einen oszillierenden Verlauf auf. Durch einen solchen oszillierenden Signalverlauf kann das charakteristische Signal einfach und sicher erkannt werden. Ein solcher oszillierender Signalverlauf, insbesondere ein oszillierender Druckverlauf, kann dabei besonders einfach von der Zugabevorrichtung erzeugt werden, und ist insbesondere auch dann einfach zu erfassen, wenn der Bereich, in welchem die Zugabevorrichtung Mittel in den extrakorporalen Kreislauf einführt, nicht abgeschlossen, sondern offen ist, z.B. während dem Betrieb des medizinischen Geräts.

Der charakteristische, vorteilhafterweise oszillierende Signalverlauf bei korrekter Funktion der Zugabevorrichtung beruht dabei vorteilhafterweise darauf, dass die Fördermenge der Zugabevorrichtung zeitlich schwankt. So ist z. B. denkbar, dass die Zugabe getaktet wird, was zu entsprechenden Druckschwankungen im extrakorporalen Kreislauf führen kann. Insbesondere wenn nur sehr geringe Mengen an Flüssigkeit infundiert werden sollen, reicht eine getaktete Zugabe üblicherweise jedoch nicht aus, um einen messbar oszillierenden Signalverlauf, z.B. in Form eines oszillierenden Druckverlaufs, zu erzeugen, über welchen die korrekte Funktion der Zugabevorrichtung zuverlässig erkannt werden könnte.

Vorteilhafterweise wird deshalb der charakteristische Signalverlauf durch eine entsprechende, insbesondere mehrfache, insbesondere oszillierende Betätigung der Zugabevorrichtung erzeugt, durch welche dem extrakorporalen Blutkreislauf abwechseln Flüssigkeit zugegeben und abgezogen wird. Hierdurch lässt sich ein klares charakteristisches Signal erzeugen, welches zuverlässig erkannt werden kann.

Vorteilhafterweise betragen die während der insbesondere mehrfachen, insbesondere oszillierenden Betätigung der Zugabevorrichtung bewegten Volumina dabei weniger als einen Milliliter. Hierdurch kann sichergestellt werden, dass die Konzentrationsschwankungen des zugegebenen Mittels klein gehalten werden. Dennoch sind hierdurch oszillierende Druckschwankungen erzeugbar, welche im Drucksignal klar erkannt werden können.

In weiterhin vorteilhafter Weise wird der Signalverlauf zur Überprüfung bzw. Überwachung der korrekten Funktion der Zugabevorrichtung zudem über mehrere Pulse, insbesondere über mehrere Perioden der Oszillation gemessen. Dies ermöglicht eine zuverlässige Erkennung des charakteristischen Signalverlaufs wie z.B. einer Oszillation im Druckverlauf. Weiterhin vorteilhafterweise erfolgt die Oszillation erfindungsgemäß mit einer zumindest über einen gewissen Zeitraum konstanten Periode.

Weiterhin vorteilhafterweise wird der charakteristische, insbesondere oszillierende Signalverlauf dabei erfindungsgemäß in dem Signal eines Drucksensors erfaßt. So kann ein charakteristischer Druckverlauf direkt und einfach erkannt werden, um die korrekte Funktion der Zugabevorrichtung zu überprüfen und/oder zu überwachen.

Alternativ kann der charakteristische, insbesondere oszillierende Signalverlauf auch in den elektrischen Parametern, insbesondere im Antriebsstrom, einer im extrakorporalen Kreislauf angeordneten Pumpe erfaßt werden, welche Flüssigkeit durch den extrakorporalen Kreislauf pumpt. Auch so lässt sich ein charakteristischer Signalverlauf zuverlässig erkennen, wobei gegebenenfalls auf einen Drucksensor verzichtet werden kann. Bei einer Veränderung des Drucks - hervorgerufen durch den veränderten Signalverlauf durch die Zugabevorrichtung - vor oder hinter der Pumpe im extrakorporalen Kreislauf muss diese z. B. mit einem höheren oder niedrigerem Strom betrieben werden. Diese Änderung der Stromaufnahme kann erfaßt werden, und im Zusammenhang mit der Einspeisung des charakteristischen Signalverlaufs ausgewertet werden. Im Fehlerfall (Infusionsleitung nicht korrekt angeschlossen oder Spritze falsch eingelegt) wird dann entsprechend kein charakteristischer Signalverlauf erfaßt.

Weiterhin vorteilhafterweise wird der charakteristische, insbesondere oszillierende Signalverlauf mittels einer Signalanalyse, insbesondere einer Frequenzanalyse erfaßt. Eine Frequenzanalyse kann dabei z. B. über bekannte Verfahren wie z. B. eine Fourier-Transformation erfolgen.

Vorteilhafterweise führt bei dem erfindungsgemäßen Verfahren die Betätigung der Zugabevorrichtung insgesamt zu einer Zugabe des Mittels in den extrakorporalen Kreislauf. Die Bestimmung der zugegebenen Menge des Mittels im zeitlichen Mittel der Bewegung der Zugabevorrichtung kann dann weiterhin wie im Stand der Technik über einen Messwertgeber an der Zugabevorrichtung geschehen, über dessen Messwerte die zugegebenen Menge indirekt bestimmt wird. Dass diese Menge auch tatsächlich im extrakorporalen Kreislauf angekommen ist, wird dagegen erfindungsgemäß über den charakteristischen Signalverlauf wie z.B. einen oszillierenden Druckverlauf erkannt.

Vorteilhafterweise erfolgt die erfindungsgemäße Überwachung der korrekten Funktion der Zugabevorrichtung dabei durchgehend während der Zugabe des Mittels in den extrakorporalen Kreislauf. Eine solche permanente Überwachung ist durch den charakteristischen und insbesondere oszillierenden Signalverlauf wie z.B. einen oszillierenden Druckverlauf, welcher auch während des laufenden Betriebs des medizinischen Gerätes erzeugt und erkannt werden kann, möglich.

In weiterhin vorteilhafter Weise erfolgt die Überprüfung und/oder Überwachung der Zugabevorrichtung dabei, während Flüssigkeit durch den extrakorporalen Kreislauf fließt, insbesondere während Flüssigkeit mittels einer im extrakorporalen Kreislauf angeordneten Pumpe durch diesen gepumpt wird. Eine solche Überprüfung bzw. Überwachung war mit bekannten Verfahren nicht möglich.

In vorteilhafter Weise erfolgt dabei die Überprüfung und/oder Überwachung der Zugabevorrichtung während des laufenden Betriebs des medizinischen Geräts. So kann die Sicherheit bei der Therapie erheblich gesteigert werden, da nun erstmals eine direkte Überwachung während des laufenden Betriebs möglich ist. Vorteilhafterweise wird der Betrieb des medizinischen Geräts unterbrochen und/oder ein Warnsignal erzeugt, wenn eine Fehlfunktion des Zugabegeräts erkannt wird. So wird sichergestellt, dass die Fehler z. B. bei der Ankopplung der Zugabevorrichtung sofort behoben werden.

Vorteilhafterweise handelt es sich bei dem zugegebenen Mittel um ein Antikoagulationsmittel, ein Medikament oder um ein anderes zu infundierendes Mittel, welches dem extrakorporalen Kreislauf zugegeben wird. In weiterhin vorteilhafter Weise handelt es sich dabei bei dem Antikoagulationsmittel um Heparin.

In weiterhin vorteilhafter Weise handelt es sich bei der erfindungsgemäßen Zugabevorrichtung um eine Spritzenpumpe mit einer darin eingesetzten Spritze. Insbesondere zur Zugabe von Antikoagulationsmittel oder anderen Medikamenten, welche nur in sehr geringen Mengen kontrolliert zugegeben werden müssen, ist eine solche Spritzenpumpe besonders gut zur Zugabe geeignet.

Vorteilhafterweise wird bei Verwendung einer Spritzenpumpe durch eine entsprechende Ansteuerung der Bewegung des Stößels der Spritzenpumpe der charakteristische, insbesondere oszillierende Signalverlauf erzeugt.

Vorteilhafterweise wird dabei der kontinuierlichen Translationsbewegung des Stößels der Spritzenpumpe, über welche dem extrakorporalen Kreislauf das Mittel zugegeben wird, eine charakteristische, insbesondere oszillierende Bewegung überlagert, welche den charakteristischen Signalverlauf und dabei insbesondere die Oszillation im Druckverlauf erzeugt.

Vorteilhafterweise werden die erfindungsgemäßen Verfahrensabläufe mittels einer Steuervorrichtung des medizinischen Geräts gesteuert. Insbesondere werden diese Verfahrensabläufe vorteilhafterweise automatisch von der Steuervorrichtung erzeugt.

Die vorliegende Erfindung umfasst weiterhin ein medizinisches Gerät, vorzugsweise eine Dialysemaschine, geeignet zur Aufnahme eines extrakorporalen Kreislaufes, mit einer Zugabevorrichtung, welche mit dem extrakorporalen Kreislauf derart verbindbar ist, dass mittels der Zugabevorrichtung ein Mittel in den extrakorporalen Kreislauf einführbar ist. Erfindungsgemäß weist dieses medizinische Gerät eine Steuervorrichtung auf, welche die Zugabevorrichtung so ansteuert, dass diese einen charakteristischen Signalverlaufs im extrakorporalen Kreislauf erzeugt, indem einer kontinuierlichen Bewegung der Zugabevorrichtung, über welche das Mittel in den extrakorporalen Kreislauf zugegeben wird, zur Erzeugung des charakteristischen, insbesondere oszillierenden Signalverlaufs eine entsprechende, insbesondere oszillierende Bewegung überlagert wird, wobei der charakteristische Signalverlauf vorzugsweise durch eine Auswertung der Steuerung erfaßbar ist. Offensichtlich ergeben sich durch ein solches medizinisches Gerät die gleichen Vorteile, insbesondere bei der Betriebssicherheit, wie sie oben im Bezug auf das Verfahren beschrieben wurden.

Vorteilhafterweise wertet die Steuerung einen Signalverlauf aus, insbesondere einen Signalverlauf im extrakorporalen Kreislauf. Durch diese Auswertung des Signalverlaufs kann ein charakteristischer Signalverlauf, wie er von der Steuerung vorteilhafterweise über die Ansteuerung der Zugabevorrichtung erzeugt wird, erfaßt werden, um die korrekte Funktion der Zugabevorrichtung zu überprüfen und/oder zu überwachen.

Weiterhin vorteilhafterweise ist die Steuerung des erfindungsgemäßen medizinischen Gerätes dabei so ausgeführt, dass sie eines der oben beschriebenen erfindungsgemäßen Verfahren durchführt. Insbesondere führt die Steuerung dabei vorteilhafterweise eines dieser Verfahren automatisch durch.

Vorteilhafterweise handelt es sich dabei bei der Zugabevorrichtung des medizinischen Geräts um eine Spritzenpumpe, in welche eine Spritze einsetzbar ist, so dass der Stempel der Spritze über einen Stößel bewegbar ist. Die Steuerung erzeugt dabei eine charakteristische, insbesondere oszillierende Bewegung des Stößels. Ist die Spritze korrekt eingelegt und konnektiert, erzeugt diese Bewegung des Stößels eine entsprechende charakteristische, insbesondere oszillierende Bewegung des Spritzenstempels, wodurch wiederum ein charakteristischer Signalverlauf, insbesondere oszillierender Druckverlauf im extrakorporalen Kreislauf entsteht. Durch die Messung dieses charakteristischen Signalverlaufs bzw. Druckverlaufs kann die korrekte Funktion der Spritzenpumpe überwacht werden, was wiederum automatisch über die Steuerung erfolgt.

Vorteilhafterweise wird dabei zusätzlich zu der Überprüfung durch das erfindungsgemäße Verfahren, welches die korrekte Funktion der Zugabe über die Spritze direkt überwacht, die Bewegung des Stößels gemessen. Über diese Bewegung des Stößels wird dann die infundierte Menge berechnet, wie es aus dem Stand der Technik bekannt ist. Vorteilhafterweise weist die Spritzenpumpe hierfür einen entsprechenden Meßwertgeber, z. B. eine Widerstandspiste auf.

In weiterhin vorteilhafter Weise weist das medizinische Gerät einen Drucksensor zur Messung des Druckverlaufs in dem extrakorporalen Kreislauf auf. Insbesondere kann es sich bei diesem Drucksensor um einen ohnehin im medizinischen Gerät vorgesehenen Drucksensor handeln, z. B. um die arterielle Druckmesseinheit.

In weiterhin vorteilhafter Weise weist das medizinische Gerät eine Pumpe zum Transport von Flüssigkeit im extrakorporalen Blutkreislauf auf.

In weiterhin vorteilhafter Weise ist die Zugabevorrichtung mit der arteriellen Leitung eines Blutschlauchsatzes für ein extrakorporales Blutbehandlungsverfahren verbindbar. Hier ist üblicher Weise auch die arterielle Druckmesseinheit vorgesehen, so dass zur Durchführung des erfindungsgemäßen Verfahrens lediglich die Steuerung des medizinischen Geräts entsprechend angepasst werden muss, ansonsten aber keine konstruktiven Veränderungen vorgenommen werden müssen.

Vorteilhafterweise handelt es sich bei dem erfindungsgemäßen medizinischen Gerät dabei um ein medizinisches Gerät zur Dialyse, Hämodialyse, Hämofiltration, Hämodiafiltration, zur adsorptiven Blutreinigung, im Transfusionsverfahren oder im Autotransfusionsverfahren. Bei all diesen medizinischen Geräten kann die erfindungsgemäße Überwachung und/oder Überprüfung der korrekten Funktion der Zugabevorrichtung die Sicherheit der Behandlung erheblich erhöhen.

In weiterhin vorteilhafter Weise umfasst die vorliegende Erfindung deshalb die Verwendung eines erfindungsgemäßen medizinischen Geräts in der Dialyse, Hämodialyse, Hämofiltration, Hämodiafiltration, zur absorptiven Blutreinigung, im Transfusionsverfahren oder im Autotransfusionsverfahren.

Die vorliegende Erfindung umfasst weiterhin eine Zugabevorrichtung, insbesondere eine Spritzenpumpe zur Zugabe einer medizinischen Flüssigkeit in eine Leitung, insbesondere in einen extrakorporalen Kreislauf oder einen Infusionsschlauch. Diese Zugabevorrichtung weist erfindungsgemäß eine Steuerung auf, welche die Zugabevorrichtung so ansteuert, dass diese einen charakteristischen Signalverlauf in der Leitung erzeugt, indem einer kontinuierlichen Bewegung der Zugabevorrichtung, über welche das Mittel in den extrakorporalen Kreislauf zugegeben wird, zur Erzeugung des charakteristischen, insbesondere oszillierenden Signalverlaufs eine entsprechende, insbesondere oszillierende Bewegung überlagert wird, wobei Mittel zum Erfassen des charakteristischen Signalverlaufs vorgesehen sind. Die erfindungsgemäße Zugabevorrichtung mit der entsprechenden Steuerung zur Überwachung und/oder Überprüfung der korrekten Funktion der Zugabevorrichtung kann damit auch unabhängig von dem erfindungsgemäßen medizinischen Gerät mit großem Vorteil eingesetzt werden. Z. B. kann die Zugabevorrichtung dabei auch zur Überwachung einer Infusion herangezogen werden.

Vorteilhafterweise ist die Zugabevorrichtung bzw. die Steuerung der Zugabevorrichtung dabei so ausgeführt, dass diese eines der oben beschriebenen Verfahren ausführt.

Die vorliegende Erfindung wird nun anhand von Zeichnungen und einem Ausführungsbeispiel näher erläutert.

Dabei zeigen:
- Figur 1:: ein Ausführungsbeispiel einer erfindungsgemäßen Zugabevorrichtung und
- Figur 2:: ein Diagramm eines Druckverlaufs während eines Ausführungsbeispiels des erfindungsgemäßen Verfahrens.

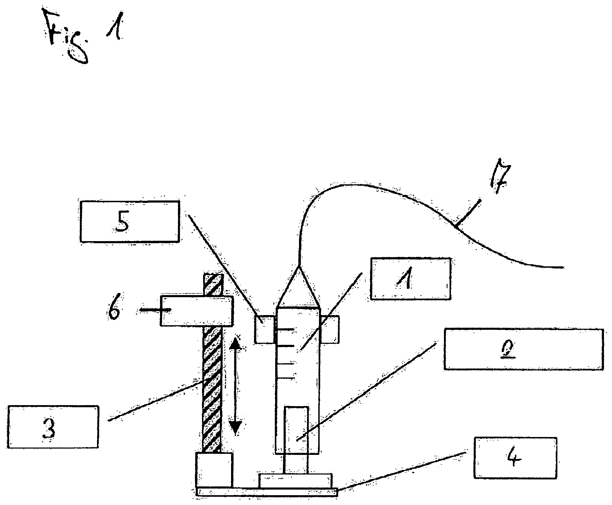

In Figur 1 ist ein Ausführungsbeispiel einer erfindungsgemäßen Zugabevorrichtung gezeigt, in diesem Fall eine Spritzenpumpe. In eine solche Spritzenpumpe wird eine Spritze 1 in eine Halterung 5 eingelegt, so dass der Spritzenstempel 2 der Spritze 1 über den Stößel 4 bewegt werden kann. Der Spritzenstempel 2 wird dabei so mit dem Stößel 4 verbunden, dass der Spritzenstempel sowohl in die Spritze hineingedrückt als auch aus der Spritze herausgezogen werden kann. Der Stößel 4 selbst wird über eine Spindel 3 vor- und zurückbewegt, welche über einen Spindelantrieb 6 angetrieben wird. Zur Abtastung der Stößelverschiebung ist zudem eine nicht gezeigte Widerstandspiste vorgesehen, so dass über die Messung des vom Förderstößel zurückgelegten Wegs die Volumenabgabe über die Spritze 1 bestimmt werden kann.

Die erfindungsgemäße Zugabevorrichtung ist dabei Teil eines erfindungsgemäßen medizinischen Geräts, insbesondere eines Dialysegeräts. In dieses medizinische Gerät ist ein extrakorporaler Kreislauf einsetzbar, insbesondere in Form eines Blutschlauchsatzes. Die Spritze 1 wird beim Einlegen dann über eine Leitung 7 mit dem extrakorporalen Kreislauf verbunden, z. B. mit der arteriellen Leitung des Blutschlauchsatzes. Über die Spritzenpumpe kann so Antikoagulationsmittel, wie z. b. Heparin oder Citrat, welches auf die Spritze 1 aufgezogen wird, kontinuierlich dem durch den extrakorporalen Kreislauf fließenden Blut zugegeben werden.

Die Steuerung des erfindungsgemäßen medizinischen Geräts ist dabei so ausgelegt, dass zunächst initial vor Beginn der Behandlung die korrekte Kopplung der Spritze an den extrakorporalen Kreislauf geprüft wird. Dies kann bereits durch das erfindungsgemäße Verfahren erfolgen, wobei zu Beginn der Behandlung jedoch auch alternative Verfahrensmöglichkeiten bekannt sind.

Zur kontinuierlichen Überwachung der korrekten Funktion der Zugabevorrichtung wird nun im erfindungsgemäßen medizinischen Gerät das erfindungsgemäße Verfahren automatisch über die Steuerung ausgeführt. Hierzu wird der kontinuierlichen Bewegung des Stößels 4 zur kontinuierlichen Infusionsgabe eine charakteristische, Bewegung überlagert, welche in dem Ausführungsbeispiel oszillierend ist. Bei korrekter Funktion aller Komponenten erzeugt diese oszillierende Bewegung des Stößels einen oszillierenden Druckverlauf im extrakorporalen Kreislauf. Die Amplitude der Druckoszillation hängt dabei von der System-Compliance und von der Größe der Stößelbewegung ab. Die während der Oszillation bewegten Volumina betragen dabei weniger als einen Milliliter und führen so nur zu minimalen Schwankungen in der Konzentration des zugegebenen Mittels im extrakorporalen Kreislauf, führen aber dennoch zu gut meßbaren und erkennbaren Druckschwankungen. Zur Überwachung der Funktion der Zugabevorrichtung wird nun mittels eines Drucksensors z. B. in der arteriellen Leitung der Druck im extrakorporalen Kreislauf gemessen und entsprechend ausgewertet. Die Identifikation der Oszillation im Druckverlauf ist dabei bei bekannter Periodendauer mit üblicher Signalanalyse machbar. Insbesondere kann dabei eine Frequenzanalyse z. B. über eine Fourier-Transformation eingesetzt werden.

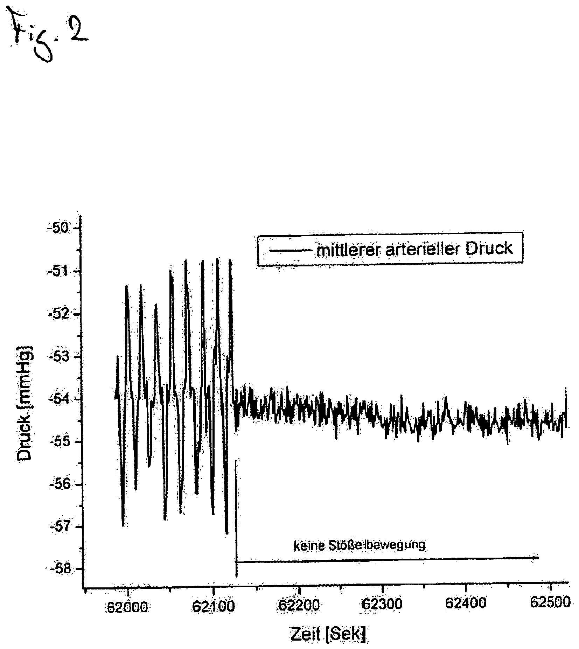

Figur 2 zeigt einen solchen Druckverlauf im mittleren arteriellen Druck. Die Zugabevorrichtung arbeitet dabei zunächst im linken Bereich des Diagramms korrekt, so dass eine Oszillation im Druckverlauf erkennbar ist. Diese wird über die Signalanalyse erkannt und ermöglicht der Steuerung des medizinischen Geräts die Überwachung der korrekten Funktion der Zugabevorrichtung. Liegt, wie im rechten Abschnitt des Diagramms, jedoch ein Fehler in der Funktion der Zugabevorrichtung vor, ist der Druckverlauf nicht mit einer Oszillation überlagert. Ein solches Signal würde sich bei einer falsch eingelegten Spritze 1 ergeben, bei welcher der Stößel 4 den Spritzenstempel 2 nicht bewegen würde. Ebenso ergäbe sich kein oszillierendes Signal im Druckverlauf, wenn keine korrekte Kopplung zwischen der Spritze 1 und dem extrakorporalen Kreislauf vorläge.

Die Steuerung des erfindungsgemäßen medizinischen Geräts erkennt nun, dass keine Oszillation im Drucksignal vorliegt. Wird ein solches Fehlen einer Oszillation erkannt, obwohl die Zugabevorrichtung betätigt wird, wird auf eine Fehlfunktion geschlossen. Das Steuergerät aktiviert dann eine Warnfunktion, z. B. über ein optisches oder akustisches Signal. So kann verhindert werden, dass es aufgrund einer Fehlfunktion der Zugabevorrichtung zu einer zu geringen Zugabe von Infusionsmitteln kommt. In einer weiteren Ausführungsform oder in besonderen Fällen kann das Steuergerät das medizinische Gerät auch automatisch stoppen.

Durch das erfindungsgemäße Verfahren bzw. die entsprechende Steuerung des erfindungsgemäßen medizinischen Gerätes ist es erstmals möglich, während dem Betrieb des medizinischen Geräts, d.h. während der eigentlichen Behandlung die korrekte Funktion der Zugabevorrichtung zu überprüfen. Insbesondere entstehen die Druckschwankungen im Druckverlauf durch die oszillierenden Bewegungen des Stößels auch dann, wenn Flüssigkeit durch den extrakorporalen Kreislauf fließt, insbesondere auch dann, wenn Blut mittels einer Blutpumpe durch den extrakorporalen Kreislauf gepumpt wird. Vorteilhafterweise ist der Drucksensor, über welchen der Druckverlauf im extrakorporalen Blutkreislauf gemessen wird, hierzu auf der gleichen Seite einer Blutpumpe angeordnet wie die Zugabevorrichtung. Vorteilhafterweise sind Zugabevorrichtung und Drucksensor dabei hintereinander auf der Saugseite einer Pumpe im extrakorporalen Kreislauf angeordnet.

Auch die elektrischen Parameter der Blutpumpe wie deren Leistungsaufnahme, Strom oder Spannung, die von den oszillierenden Druckpulsen beeinflusst werden, können zur Detektion und Auswertung herangezogen werden.

Die kontinuierliche Überwachung der Infusionsfördermenge besteht hierdurch einerseits aus der Sicherstellung der korrekten Funktion der Zugabevorrichtung, insbesondere der korrekten Kopplung zwischen Spritze 1 und Stößel 4 sowie der korrekten Kopplung der Spritze 1 an den extrakorporalen Kreislauf durch die vorliegende Erfindung.

Zudem umfasst die Überwachung weiterhin eine Messung der innerhalb einer bestimmten Zeit verabreichten Infusionsmenge. Dies wird dabei über die Abtastung der Stößelverschiebung mit seiner Widerstandspiste wie im Stand der Technik realisiert.

Durch die Sicherstellung der permanenten Ankopplung mit Hilfe des erfindungsgemäßen Verfahrens können nun erstmals Probleme im Zusammenhang mit der Infusion, welche sich erst während der Behandlung ergeben und zu einer Abweichung der tatsächlich verabreichten von der gemessenen Infusionsgabe führen würden, zuverlässig erkannt werden.

## Patentansprüche

1. Verfahren zur Überprüfung und/oder Überwachung der korrekten Funktion einer Zugabevorrichtung eines medizinischen Geräts, wobei das medizinische Gerät einen extrakorporalen Kreislauf aufweist, mit dem die Zugabevorrichtung derart in Verbindung steht, dass mittels der Zugabevorrichtung ein Mittel in den extrakorporalen Kreislauf einführbar ist, wobei ein charakteristischer Signalverlauf im extrakorporalen Kreislauf erfaßt wird, wobei die Zugabevorrichtung den charakteristischen Signalverlauf erzeugt,
**dadurch gekennzeichnet,**
**dass** einer kontinuierlichen Bewegung der Zugabevorrichtung, über welche das Mittel in den extrakorporalen Kreislauf zugegeben wird, zur Erzeugung des charakteristischen, insbesondere oszillierenden Signalverlaufs eine entsprechende, insbesondere oszillierende Bewegung überlagert wird.

2. Verfahren nach Anspruch 1, wobei der charakteristische Signalverlauf ein charakteristischer Druckverlauf ist, bei dem der charakteristische Signalverlauf vorzugsweise ein mehrmaliges abwechselndes Ansteigen und Abfallen des Signals, insbesondere mehrere aufeinanderfolgende pulsförmige Signale, und gegebenenfalls einen oszillierenden Verlauf aufweist.

3. Verfahren nach einem der vorangegangenen Ansprüche, wobei der charakteristische Signalverlauf durch eine entsprechende, insbesondere mehrfache, insbesondere oszillierende Betätigung der Zugabevorrichtung erzeugt wird, bei welcher dem extrakorporalen Kreislauf abwechselnd Flüssigkeit vorzugsweise mit Volumina von weniger als 1 ml zugegeben und abgezogen wird.

4. Verfahren nach einem der vorangegangenen Ansprüche, wobei der Signalverlauf über mehrere Pulse, insbesondere über mehrere Perioden der Oszillation gemessen wird.

5. Verfahren einem der vorangegangenen Ansprüche, wobei der charakteristische, insbesondere oszillierende Signalverlauf in dem Signal eines Drucksensors oder in den elektrischen Parametern, insbesondere im Antriebsstrom, einer im extrakorporalen Kreislauf angeordneten Pumpe erfaßt wird, welche Flüssigkeit durch den extrakorporalen Kreislauf pumpt.

6. Verfahren einem der vorangegangenen Ansprüche, wobei der charakteristische, insbesondere oszillierende Signalverlauf mittels einer Signalanalyse, insbesondere einer Frequenzanalyse, erfaßt wird.

7. Verfahren nach einem der vorangegangenen Ansprüche, wobei die Betätigung der Zugabevorrichtung insgesamt zu einer Zugabe des Mittels in den extrakorporalen Kreislauf führt, während die Überwachung der korrekten Funktion der Zugabevorrichtung durchgehend während der Zugabe des Mittels in den extrakorporalen Kreislauf erfolgt und/oder während die Überprüfung und/oder Überwachung der Zugabevorrichtung erfolgt, während Flüssigkeit durch den extrakorporalen Kreislauf fließt, insbesondere während Flüssigkeit mittels einer im extrakorporalen Kreislauf angeordneten Pumpe durch diesen gepumpt wird und/oder während die Überprüfung und/oder Überwachung der Zugabevorrichtung während des laufenden Betriebs des medizinischen Geräts erfolgt, wobei bevorzugt der Betrieb des medizinischen Geräts unterbrochen und/oder ein Warnsignal erzeugt wird, wenn eine Fehlfunktion der Zugabevorrichtung erkannt wird.

8. Verfahren nach einem der vorhergehenden Ansprüche, **dadurch gekennzeichnet, dass** es sich bei dem in zugegebenen Mittel um ein Antikoagulationsmittel, vorzugsweise Heparin, ein Medikament oder ein anderes zu infundierendes Mittel handelt.

9. Verfahren nach einem der vorangegangenen Ansprüche, wobei es sich bei der Zugabevorrichtung um eine Spritzenpumpe mit einer darin eingesetzten Spritze (1) handelt, wobei der charakteristische, insbesondere oszillierende Signalverlauf durch eine entsprechende Ansteuerung der Bewegung des Stößels (4) der Spritzenpumpe erzeugt wird, indem bevorzugt der kontinuierlichen Translationsbewegung des Stößels (4) der Spritzenpumpe zur Erzeugung des charakteristischen, insbesondere oszillierenden Signalverlaufs eine entsprechende charakteristische, insbesondere oszillierende Bewegung überlagert wird und wobei die Verfahrensabläufe vorzugsweise mittels einer Steuervorrichtung des medizinischen Geräts gesteuert werden.

10. Medizinisches Gerät, vorzugsweise Dialysemaschine, zur Durchführung eines Verfahrens nach einem der Ansprüche 1 bis 9 geeignet zur Aufnahme eines extrakorporalen Kreislaufes, mit einer Zugabevorrichtung, welche mit dem extrakorporalen Kreislauf derart verbindbar ist, dass mittels der Zugabevorrichtung ein Mittel in den extrakorporalen Kreislauf einführbar ist,
**dadurch gekennzeichnet,**
**dass** das medizinische Gerät eine Steuerung aufweist, welche die Zugabevorrichtung so ansteuert, dass diese einen charakteristischen Signalverlauf im extrakorporalen Kreislauf erzeugt, indem einer kontinuierlichen Bewegung der Zugabevorrichtung, über welche das Mittel in den extrakorporalen Kreislauf zugegeben wird, zur Erzeugung des charakteristischen, insbesondere oszillierenden Signalverlaufs eine entsprechende, insbesondere oszillierende Bewegung überlagert wird, wobei der charakteristische Signalverlauf vorzugsweise durch eine Auswertung der Steuerung erfaßbar ist.

11. Medizinisches Gerät nach Anspruch 10, mit einer Steuerung zur Durchführung, wobei es sich bei der Zugabevorrichtung vorzugsweise um eine Spritzenpumpe handelt, in welche eine Spritze (1) einsetzbar ist, so dass der Stempel (2) der Spritze über einen Stößel (4) bewegbar ist, wobei vorzugsweise ein Drucksensor zur Messung des Druckverlaufs in dem extrakorporalen Kreislauf vorgesehen ist und wobei vorzugsweise die Zugabevorrichtung mit der arteriellen Leitung des Blutschlauchsatzes für ein extrakorporales Blutbehandlungverfahren verbindbar ist.

12. Medizinisches Gerät nach einem der vorhergehenden Ansprüche, **dadurch gekennzeichnet, dass** es sich um ein medizinische Gerät zur Dialyse, Hämodialyse, Hämofiltration, Hämodiafiltration, zur adsorptiven Blutreinigung, im Transfusionsverfahren oder im Autotransfusionsverfahren handelt.

13. Zugabevorrichtung, insbesondere Spritzenpumpe, zur Verwendung in einem Verfahren nach einem der Ansprüche 1 bis 9, zur Zugabe einer medizinischen Flüssigkeit in eine Leitung, insbesondere einen extrakorporalen Kreislauf oder einen Infusionsschlauch,
**gekennzeichnet durch**
eine Steuerung, welche die Zugabevorrichtung so ansteuert, dass diese einen charakteristischen Signalverlauf in der Leitung erzeugt, indem einer kontinuierlichen Bewegung der Zugabevorrichtung, über welche das Mittel in den extrakorporalen Kreislauf zugegeben wird, zur Erzeugung des charakteristischen, insbesondere oszillierenden Signalverlaufs eine entsprechende, insbesondere oszillierende Bewegung überlagert wird, wobei Mittel zum Erfassen des charakteristischen Signalverlaufs vorgesehen sind.

## Claims

1. A method for checking and/or monitoring the correct operation of an adding device of a medical apparatus, wherein the medical apparatus includes an extracorporeal circuit with which the adding device is connected such that an agent can be introduced into the extracorporeal circuit by means of the adding device, wherein a characteristic signal course is detected in the extracorporeal circuit, the characteristic signal course being generated by the adding device,
**characterized in**
**that** on a continuous movement of the adding device, by means of which the agent is added into the extracorporeal circuit, a corresponding, in particular oscillating movement is superimposed in order to generate the characteristic, in particular oscillating signal course.

2. The method according to claim 1, wherein the characteristic signal course is a characteristic pressure course in which the characteristic signal course preferably includes a repeated alternating rise and fall of the signal, in particular a plurality of successive pulse-shaped signals, and possibly an oscillating course.

3. The method according to any of the preceding claims, wherein the characteristic signal course is generated by a corresponding, in particular repeated, in particular oscillating actuation of the adding device, in which fluid alternately is added to and withdrawn from the extracorporeal circuit, preferably in volumes of less than 1 ml.

4. The method according to any of the preceding claims, wherein the signal course is measured over several pulses, in particular over several periods of the oscillation.

5. The method according to any of the preceding claims, wherein the characteristic, in particular oscillating signal course is detected in the signal of a pressure sensor or in the electrical parameters, in particular in the drive current of a pump arranged in the extracorporeal circuit, which pumps fluid through the extracorporeal circuit.

6. The method according to any of the preceding claims, wherein the characteristic, in particular oscillating signal course is detected by means of a signal analysis, in particular a frequency analysis.

7. The method according to any of the preceding claims, wherein the actuation of the adding device on the whole leads to an addition of the agent into the extracorporeal circuit, while the monitoring of the correct operation of the adding device is effected continuously during the addition of the agent into the extracorporeal circuit and/or during the checking and/or monitoring of the adding device, while fluid flows through the extracorporeal circuit, in particular while fluid is pumped through the same by means of a pump arranged in the extracorporeal circuit and/or during the checking and/or monitoring of the adding device during the ongoing operation of the medical apparatus, wherein preferably the operation of the medical apparatus is interrupted and/or a warning signal is generated when a malfunction of the adding device is detected.

8. The method according to any of the preceding claims, **characterized in that** the agent added is an anticoagulant, preferably heparin, a drug or some other agent to be infused.

9. The method according to any of the preceding claims, wherein the adding device is a syringe pump with a syringe (1) inserted therein, wherein the characteristic, in particular oscillating signal course is generated by a corresponding actuation of the movement of the tappet (4) of the syringe pump, in that preferably a corresponding characteristic, in particular oscillating movement is superimposed on the continuous translational movement of the tappet (4) of the syringe pump in order to generate the characteristic, in particular oscillating signal course, and wherein the processes preferably are controlled by means of a control device of the medical apparatus.

10. A medical apparatus, preferably a dialysis machine, for carrying out a method according to any of claims 1 to 9 suitable for accommodating an extracorporeal circuit, comprising an adding device that is connectable with the extracorporeal circuit such that by means of the adding device an agent can be introduced into the extracorporeal circuit,
**characterized in**
**that** the medical apparatus includes a control unit which actuates the adding device such that the same generates a characteristic signal course in the extracorporeal circuit, in that on a continuous movement of the adding device, via which the agent is added into the extracorporeal circuit, a corresponding, in particular oscillating movement is superimposed in order to generate the characteristic, in particular oscillating signal course, wherein the characteristic signal course preferably is detectable by an evaluation of the control unit.

11. The medical apparatus according to claim 10, comprising a control unit for performing, wherein the adding device preferably is a syringe pump into which a syringe (1) can be inserted, so that the stamp (2) of the syringe is movable via a tappet (4), wherein preferably a pressure sensor is provided for measuring the pressure course in the extracorporeal circuit, and wherein preferably the adding device can be connected to the arterial line of the blood tubing set for an extracorporeal blood treatment method.

12. The medical apparatus according to any of the preceding claims, **characterized in that** it is a medical apparatus for the dialysis, hemodialysis, hemofiltration, hemodiafiltration, for the adsorptive blood cleaning, by a transfusion method or by an autotransfusion method.

13. An adding device, in particular a syringe pump, for use in a method according to any of claims 1 to 9, for the addition of a medical fluid into a line, in particular an extracorporeal circuit or an infusion tube,
**characterized by**
a control unit which actuates the adding device such that the same generates a characteristic signal course in the line, in that on a continuous movement of the adding device, by which the agent is added into the extracorporeal circuit, a corresponding, in particular oscillating movement is superimposed in order to generate the characteristic, in particular oscillating signal course, wherein means for detecting the characteristic signal course are provided.

## Revendications

1. Procédé pour contrôler et/ou surveiller le fonctionnement correct d'un dispositif d'ajout d'un appareil médical, l'appareil médical comprenant un circuit extracorporel auquel le dispositif d'ajout est relié de sorte qu'au moyen du dispositif d'ajout un agent peut être introduit dans le circuit extracorporel, un cours de signal caractéristique étant détecté dans le circuit extracorporel, le dispositif d'ajout générant le cours de signal caractéristique,
**caractérisé en ce que**
sur un mouvement continu du dispositif d'ajout, par lequel l'agent est ajouté dans le circuit extracorporel, un mouvement correspondant, en particulier oscillant est superposé pour générer le cours de signal caractéristique, in particulier oscillant.

2. Procédé selon la revendication 1, le cours de signal caractéristique étant un cours de pression caractéristique, dans lequel le cours de signal caractéristique de préférence présente une montée et descente du signal alternante plusieurs fois, en particulier plusieurs signaux successifs en forme d'impulsions, et éventuellement un cours oscillant.

3. Procédé selon l'une quelconque des revendications précédentes, le cours de signal caractéristique étant généré par un actionnement correspondant, en particulier multiple, en particulier oscillant du dispositif d'ajout, dans lequel du fluide est ajouté et retiré en alternance du circuit extracorporel en volumes de moins de 1 ml.

4. Procédé selon l'une quelconque des revendications précédentes, le cours de signal étant mesuré sur plusieurs impulsions, en particulier sur plusieurs périodes de l'oscillation.

5. Procédé selon l'une quelconque des revendications précédentes, le cours de signal caractéristique, en particulier oscillant étant détecté dans le signal d'un capteur de pression ou dans les paramètres électriques, en particulier dans le courant d'entraînement, d'une pompe agencée dans le circuit extracorporel, qui pompe du fluide à travers le circuit extracorporel.

6. Procédé selon l'une quelconque des revendications précédentes, le cours de signal caractéristique, en particulier oscillant étant détecté au moyen d'une analyse de signal, en particulier d'une analyse de fréquence.

7. Procédé selon l'une quelconque des revendications précédentes, l'actionnement du dispositif d'ajout en général conduisant à un ajout de l'agent dans le circuit extracorporel, tandis que la surveillance du fonctionnement correct du dispositif d'ajout est effectuée en continu pendant l'ajout de l'agent dans le circuit extracorporel et/ou pendant le contrôle et/ou surveillance du dispositif d'ajout, pendant du fluide s'écoule à travers le circuit extracorporel, en particulier pendant du fluide est pompé à travers le circuit extracorporel au moyen d'une pompe agencée dans celui-ci et/ou pendant le contrôle et/ou surveillance du dispositif d'ajout pendant le fonctionnement en cours de l'appareil médical, le fonctionnement de l'appareil médical de préférence étant interrompu et/ou un signal d'alarme étant généré, quand un mauvais fonctionnement du dispositif d'ajout est détecté.

8. Procédé selon l'une quelconque des revendications précédentes, **caractérisé en ce que** l'agent ajouté est un anticoagulant, de préférence de l'héparine, un médicament ou un autre agent à perfuser.

9. Procédé selon l'une quelconque des revendications précédentes, le dispositif d'ajout étant une pompe à seringue avec une seringue (1) insérée dedans, le cours de signal caractéristique, en particulier oscillant étant généré par un actionnement correspondant du mouvement du poussoir (4) de la pompe à seringue en superposant de préférence sur le mouvement de translation continu du poussoir (4) de la pompe à seringue un mouvement correspondant caractéristique, en particulier oscillant pour générer le cours de signal caractéristique, en particulier oscillant, et les procédures de préférence étant commandées au moyen d'un dispositif de commande de l'appareil médical.

10. Appareil médical, de préférence machine de dialyse, pour la réalisation d'un procédé selon l'une quelconque des revendications 1 à 9, adapté à recevoir un circuit extracorporel, comportant un dispositif d'ajout, qui peut être relié au circuit extracorporel de sorte qu'au moyen du dispositif d'ajout un agent peut être introduit dans le circuit extracorporel,
**caractérisé en ce que**
l'appareil médical possède une unité de commande, qui actionne le dispositif d'ajout de sorte que celui-ci génère un cours de signal caractéristique dans le circuit extracorporel en superposant sur un mouvement continu du dispositif d'ajout, par lequel l'agent est ajouté dans le circuit extracorporel, un mouvement correspondant, en particulier oscillant, pour générer le cours de signal caractéristique, en particulier oscillant, le cours de signal caractéristique de préférence étant détectable par un évaluation de l'unité de commande.

11. Appareil médical selon la revendication 10, comportant une unité de commande pour la réalisation, le dispositif de commande de préférence étant une pompe à seringue dans laquelle une seringue (1) peut être insérée de sorte que le piston (2) de la seringue peut être déplacé par un poussoir (4), un capteur de pression de préférence étant prévu pour mesurer le cours de pression dans le circuit extracorporel, et le dispositif d'ajout de préférence pouvant être relié à la ligne artérielle de l'ensemble de tubulures pour le sang pour un procédé de traitement du sang extracorporel.

12. Appareil médical selon l'une quelconque des revendications précédentes, **caractérisé en ce qu'**il s'agit d'un appareil médical pour la dialyse, l'hémodialyse, l'hémofiltration, l'hémodiafiltration, la purification adsorptive du sang, par un procédé de transfusion ou un procédé d'autotransfusion.

13. Dispositif d'ajout, en particulier pompe à seringue, à utiliser dans un procédé selon l'une quelconque des revendications 1 à 9, pour ajouter un fluide médical dans une ligne, en particulier un circuit extracorporel ou un tube de perfusion,
**caractérisé par**
une unité de commande, qui actionne le dispositif d'ajout de sorte que celui-ci génère un cours de signal caractéristique dans la ligne en superposant sur un mouvement continu du dispositif d'ajout, par lequel l'agent est ajouté dans le circuit extracorporel, un mouvement correspondant, en particulier oscillant, pour générer le cours de signal caractéristique, en particulier oscillant, des moyens pour détecter le cours de signal caractéristique étant prévus.
